# EUROPÄISCHE PATENTSCHRIFT

(11) **EP 2 819 711 B1**
(45) Veröffentlichungstag und Bekanntmachung des Hinweises auf die Patenterteilung: **11.11.2020**
(21) Anmeldenummer: 13711315.5
(22) Anmeldetag: 01.03.2013
(51) Int. Cl.: A61L 15/28, A61L 15/46

(54) **ANTISEPTISCHE WUNDAUFLAGE**
ANTISEPTIC WOUND DRESSING
PANSEMENT ANTISEPTIQUE

(30) Priorität: 02.03.2012 DE 102012004024
(43) Veröffentlichungstag der Anmeldung: 07.01.2015
(73) Patentinhaber: Hawest Research AG, 6312 Steinhausen (CH)
(72) Erfinder: HORCHLER, Harald K., 51674 Wiehl (DE); BUSCHMANN, Hans-Jürgen, 47906 Kempen (DE)
(74) Vertreter: Thiel, Christian
(86) Internationale Anmeldenummer: PCT/EP2013/054132
(87) Internationale Veröffentlichungsnummer: WO 2013/127983

(56) Entgegenhaltungen:
- US-A1- 2007 207 189
- Patching J W et al.: "Industrial biofilms: formation, problems and control" In: LENS P et al.: "Biofilms in Medicine, Industry and Environmental Biotechnology", 1 January 2003 (2003-01-01), IWA Publishing, London, UK, XP055262465, ISBN: 978-1-84339-019-0 page 579,
- SEIPP H M ET AL: "Efficacy of various wound irrigation solutions against biofilms - Wirksamkeit verschiedener Wundspüllösungen gegenüber Biofilmen", ZEITSCHRIFT FUER WUNDHEILUNG - WUNDMANAGEMENT, M H P VERLAG GMBH, DE, vol. 10, no. 4, 1 January 2005 (2005-01-01), pages 160-164, XP008184880, ISSN: 1439-670X

## Beschreibung

Die Erfindung betrifft eine antiseptische Wundauflage mit einem Trägermaterial und einem oder mehreren Antiseptika und einem Tensid.

Antiseptisch ausgerüstete Materialien für den Klinikbereich, insbesondere für die Wundpflege, aber auch für kosmetische und sonstige Zwecke sind weithin bekannt und im Einsatz. Solche Materialien bestehen in der Regel aus einem Trägermaterial, das mit einem oder mehreren Antiseptika ausgerüstet ist. Als Trägermaterial kommen sowohl textile Materialien als auch nicht-textile Materialien, etwa Polymerfolien und -schäume in Frage.

Insbesondere im Bereich Krankenpflege und Wundversorgung haben sich Antiseptika auf Basis von Polyaminen und Biguaniden bewährt. Zu nennen sind beispielsweise Chlorhexidin, Polyethylenemin (Lupasol®), Spermin, Triethylentetramin oder auch Polyhexanid (PHMB, Poly(hexamethylenbiguanid)hydrochlorid). Insbesondere Chlorhexidin und PHMB haben ein erhebliches antibakterielles Potential, wobei PHMB allgemein eine geringere Toxizität auf menschliche Zellen ausübt.

Insbesondere bei der Wundversorgung, aber auch bei der antibakteriellen Ausstattung von Textilien für die Krankenversorgung und sonstige Zwecke, besteht allerdings das Problem, das die antibakterielle Wirkung der Wirkstoffe sich nach relativ kurzer Zeit erschöpft. Grund hierfür ist die geringe Dauerhaftigkeit der Wirkstoffe auf den textilen Materialien. Bei der Wundversorgung kommt hinzu, dass die Wirkstoffe durch das Wundexsudat verdünnt und mit dem Wundexsudat zusammen entfernt (abgesaugt) werden. Dies macht einen häufigen Wechsel der Wundauflage erforderlich, was der Wundheilung nicht unbedingt förderlich ist.

Ein besonderes Problem besteht bei Wunden, die mit einem saugenden Material gefüllt, verschlossen und mittels einer Pumpe oder Saugflasche unter Vakuum gesetzt werden. Es hat sich gezeigt, dass ein geringes Vakuum den Heilungsprozess fördert. Ein Imprägnierung des saugenden Materials mit einem Antiseptikum wäre hier vorteilhaft, jedoch wird dieses in aller Regel binnen ganz kurzer Zeit mit dem Exsudat aus der Wunde herausgesaugt. In der Folge droht die Wunde zu verkeimen, wenn nicht regelmäßig ein Verbandswechsel stattfindet. Ein Verbandswechsel ist allerdings dem Heilungsprozess abträglich. Eine Verlängerung der Standzeit/Wirkungsdauer der antiseptischen Ausrüstung der Wundauflage wäre hier sehr wünschenswert.

Hierzu schlägt die US 2007/0207189 A1 die Verwendung eines Cyclodextrins als micellenbildende Komponente zusammen mit einem antimikrobiell wirksamen Stoff vor. Die Wirksamkeit dieser bekannten Kombination ist jedoch nicht ausreichend für Wundauflagen.

Aufgabe der Erfindung ist in diesem Sinne die Bereitstellung einer antiseptischen Wundauflage, die ihre Wirksamkeit über längere Zeit beibehält, gleichzeitig aber wenig toxisch auf menschliche und tierische Zellen wirkt, einfach herzustellen ist und einfach zu handhaben ist.

Diese Aufgabe wird mit einer antiseptischen Wundauflage der eingangs genannten Art gelöst, bei der das Trägermaterial mit einem Cyclodextrin oder Cyclodextrinderivat ausgerüstet ist, das mit dem Antiseptikum oder mit mehreren Antiseptika beladen ist, und das Tensid ein Betaïn ist, wobei das Cyclodextrin oder Cyclodextrinderivat chemisch an ein Polysaccharid, ein Polyvinylamin oder ein Polyethylenimin gebunden ist, das seinerseits über physikalische Wechselwirkungen an das Trägermaterial gebunden ist.

Unter einer antiseptischen Wundauflage wird erfindungsgemäß eine jede Wundauflage verstanden, die auf die beschriebene Art und Weise antiseptisch ausgestattet ist. Eine solche Wundauflage weist jeweils ein Trägermaterial auf, das ein Antiseptikum und ein Tensid enthält. Als Trägermaterial kommen insbesondere textile Materialien in Frage, wobei es sich um textile Gewebe oder um Vliesstoffe handeln kann. In Frage kommen als Trägermaterial auch Kunststoffe, insbesondere poröse Kunststoffschäume, wie sie in der Medizin in der Wundbehandlung Anwendung finden, insbesondere offenporige Polyurethane, die als ausgesprochen körperfreundlich gelten.

Als Antiseptika kommen insbesondere Polyamine und Biguanide in Frage, wobei der Begriff "Polyamin" sowohl antiseptisch wirkende mehrfunktionelle Amine wie auch polymere Amine einschließt. Bevorzugt sind Biguanide, darunter Chlorhexidin und Polyhexanid.

Die erfindungsgemäß verwandten Trägermaterialien sind mit einem Cyclodextrin oder Cyclodextrinderivat ausgerüstet. Unter Cyclodextrinen werden die bekannten Formen α-, β- und γ- Cyclodextrin verstanden, wobei es sich um cyclische Zuckermoleküle mit 6, 7 oder 8 α-D-Glukoseeinheiten handelt. Diese Cyclodextrine können in üblicher Weise derivatisiert sein. Sie können ferner mit an und für sich bekannten Ankergruppen ausgestattet sein, über die eine Anbindung an ein Trägermaterial stattfindet. Diese Anbindung kann auf chemische Art und Weise erfolgen, auf physikalische Weise über Wechselwirkungen oder aber rein mechanisch über sogenannte Ankergruppen, die sich mit dem Trägermaterial verhaken. Eine physikalische Anbindung erfolgt beispielsweise über elektrostatische Wechselwirkungen oder über chemische Wechselwirkungen zwischen den Molekülen oder seines Derivats, beispielsweise hydrophile/hydrophobe Wechselwirkungen des Cyclodextrins mit der Umgebung und der Oberfläche des Trägermaterials. Alle diese Verfahren sind in der Literatur hinreichend beschrieben.

Unter Cyclodextrinderivaten werden Derivate des α-, β- und γ- Cyclodextrins verstanden, die zusätzlich zu einer Ankergruppe eine weitere Funktionalisierung aufweisen, um erwünschte Effekte zu erzielen. Solche Derivate sind so auszuwählen, dass sie keinen negativen, ggf. aber einen positiven Einfluss auf die Komplexbildung des Cyclodextrins mit dem Antiseptikum ausüben.

Als Tensid kommen übliche Tenside in Frage, insbesondere aber Betaine. Zu nennen wären hier insbesondere Undecylenamidopropylbetaïn und Trimethyammoniumacetat. Die Tenside sind in der Regel nicht an das Cyclodextrin gebunden, sondern über elektrostatische Wechselwirkungen an das Trägermaterial.

Das erfindungsgemäß zum Einsatz kommende Trägermaterial enthält in der Regel 1 bis 5 Gew.-% Cyclodextrin oder -derivat, insbesondere etwa 3 Gew.-%, bezogen auf das Gewicht des Trägermaterials. Untersuchungen haben gezeigt, dass bei richtiger Verfahrensführung im Regelfall nahezu alle Cyclodextrineinheiten mit einem Molekül des Antiseptikums beladen sind, d. h. das Verhältnis der Cyclodextrinmoleküle zu den Molekülen des Antiseptikums liegt im Bereich von 0,1 bis 1,0. Was das Tensid oder Betaïn anbetrifft, ist es in der Regel etwa in der gleichen Menge im antiseptischen Material enthalten wie das Antiseptikum.

Untersuchungen haben gezeigt, dass die erfindungsgemäße antiseptische Wundauflage eine hohe bakterizide Wirksamkeit aufweist und zudem antiviral und fungizid wirksam ist. Die fungizide Wirksamkeit ergibt sich vor allem durch den Zusatz des Betaïns, das geeignet ist, Biofilme von Wundoberflächen abzulösen und das Eindringen des Antiseptikums in die Wunde zu fördern. Es tritt eine Art von Schleppwirkung auf. Tests mit pathogenen Keimen, insbesondere aus S. Aureus, E. coli und C. albicans haben in kurzer Zeit eine praktisch 100 %ige Inhibierung des Mikroorganismus ergeben.

Hinzu kommt, dass Cyclodextrine geeignet sind, Eiweisstoffe und andere Zerfallsprodukte von Körperzellen in sich aufzunehmen und der Umgebung zu entziehen. Hieraus ergibt sich eine heilungsfördernde wie zugleich auch geruchsmindernde Wirkung. Die Wunde wird insgesamt sauberer gehalten. Mit der Aufnahme solcher Zerfallsprodukte verbunden ist die Freisetzung von im Cyclodextrin enthaltenen Antiseptika, die auf diese Art und Weise nach und nach in den Wundbereich abgegeben werden. Dies ist verantwortlich für die nachhaltige Wirkung der erfindungsgemäßen antiseptischen Wundauflage.

Besonders bevorzugte antiseptische Wundauflagen haben ein Trägermaterial, das mit β-Cyclodextrin ausgerüstet ist, welches mit Polyhexanid beladen ist. Als Betaïn ist Undecylenamidopropylbetain zugegen. Das Cyclodextrin ist dabei über eine Ankergruppe an ein Polysaccharid gebunden, welches auf das Trägermaterial aufgebracht wird. Als Polysaccharid kommen insbesondere übliche gelbildende Polysaccharide in Frage, wie Carrageenan, Tragant, Guar, Fucoidan oder Alginat. Die Anbindung des Cyclodextrins erfolgt durch Reaktion von Monochlortriazenyl-β-Cyclodextrin in Wasser bei einer erhöhten Temperatur (90°C) in Gegenwart von Natronlauge bei einem pH-Wert von 11 bis 12. Das so erhaltene Produkt, bei denen das β-Cyclodextrin an das Polysaccharid gebunden ist, kann durch Aufsprühen oder Foulardieren dauerhaft auf das Trägermaterial aufgebracht werden. Es verbleibt dort auf nach mehreren Wäschen.

Die Beladung des Cyclodextrins mit dem Antiseptikum erfolgt durch Eintauchen des Trägermaterials in eine Lösung des Antiseptikums, durch Aufsprühen des Antiseptikums oder durch Umsetzung des Monochlortriazenyl-β-Cyclodextrins mit dem Polysaccharid in Gegenwart des Antiseptikums hergestellt. In letzterem Fall muss sichergestellt sein, dass es nicht zu unerwünschten Nebenreaktionen kommt.

Eine weitere bevorzugte Form der Bindung des Cyclodextrins an das Trägermaterial kann über Polyvinylamin oder Polyethylenemin erfolgen. β-Cyclodextrin substituiertes Polyvinylamin oder Polyethylenemin haftet ausgezeichnet an der Oberfläche von Textilien, aber auch von Polyurethanschäumen, weshalb sich auch entsprechend ausgerüstetes Trägermaterial besonders für die Wundbehandlung eignet.

Entsprechend betrifft die Erfindung auch eine wässerige Lösung, die ein an ein Polyvinylamin oder Polyethylenimin gebundenes Cyclodextrin enthält, das mit Polyhexamid beladen ist und zusätzlich Undecylenamidopropylbetaïn enthält.

Tests haben gezeigt, dass ein Trägermaterial, das mit 3 % Polyvinylamin-β-Cyclodextrin ausgerüstet ist und mit in etwa gleichen Mengen PHMB und Undecylenamidopropylbetaïn beladen ist, im Folgenden als PHMB/Betaïn-Komplex oder Komplex bezeichnet eine Reihe von vorteilhaften Eigenschaften hat:
- Der PHMB/Betaïn-Komplex hat kationische Eigenschaften und bindet ausgezeichnet an OH-Bindungen, wie sie beispielsweise in Zellulose vorliegen. Eine wässrige Lösung des entsprechend beladenen Polyvinylamin-β-Cyclodextrinkomplexes kann entsprechend zur Dekontamination und Infektionsprophylaxe eingesetzt werden.
- Der Komplex zeigt einen sehr guten Biokompatibilitätsindex mit einer hohen "Killing Rate" bei Mikroorganismen und einer niedrigen Toxizität bei humanen Zellen (getestet an HaCaT-Zellen). Gezeigt hat sich eine beschleunigte Wundheilung und eine sehr geringe Infektionsrate.
- Der Komplex ist in der Lage, das pH-Milieu einer Wunde bei 5,5 zu stabilisieren und damit Voraussetzungen zur Wundkonditionierung zu schaffen. Bei chronischen Wunden liegt der pH-Wert regelmäßig im alkalischen Bereich. Bei pH 5,5 verläuft die Wundheilung deutlich schneller.
- Die Kombination aus PHMB und Betaïn löst Biofilme und reinigt damit die Wunde. Sie schafft ein optimales Wundmilieu zur schnelleren Neubildung von Granulationsgewebe.
- Der Komplex inhibiert nachhaltig freie Radikale, die geeignet sind, Zellen zu schädigen.
- Der Komplex ist in der Lage, Exsudat, insbesondere Proteine aus der Wunde aufzunehmen und zu neutralisieren. Damit wird Mikroorganismen die Nahrungsgrundlage entzogen.

### Beispiele

Die Erfindung wird durch die nachfolgenden Beispiele näher beschrieben.

Die in den vorliegenden Beispielen hergestellten Lösungen sind zum Einsprühen von textilen Materialien, Schäumen, etc. aus den unterschiedlichsten Materialien, wie z.B. Polyester, Baumwolle, Polyacrylnitril, Glasfasern, Polyurethan usw. geeignet. Die Fixierung der Cyclodextrinderivate erfolgt durch elektrostatische und hydrophobe Wechselwirkungen mit der jeweiligen Oberfläche.

Die angegebenen Konzentrationen dürfen nicht wesentlich überschritten werden, da sonst eine starke Gelbildung einsetzt und Reaktionslösung nicht mehr auf textile Materialien aufgesprüht werden kann.

### Beispiel 1

### Umsetzung mit Carrageenan (Fluka)

1g Carrageenan wird zusammen mit 2g Monochlortriazenyl-β-Cyclodextrin (Wacker) in 200ml Wasser gelöst. Die Lösung wird auf 90°C erhitzt und es wird langsam eine Lösung von Natronlauge (15g/l) zugetropft, bi ein pH-Wert von 11 bis 12 eingestellt ist. Nach einer Stunde wird die Lösung abgekühlt und mit Salzsäure neutralisiert.

Entsprechende Cyclodextrinderivate wurden mit den folgenden Polysachariden hergestellt:
Tragant (Merck)
Guar (Sigma)
Fucoidan (Sigma)
Carrageenan/Fucoidan

Die in diesem Beispiel synthetisierten Cyclodextrinderivate besitzen keinerlei biostatische Effekte. Die biostatische Wirkung der Ausrüstung kann mit Hilfe des Formazantests (F.P. Altman, Progr. Histochem. Cytochem. 9 (1976); W. Oppermann, R. Gutmann, S. Schmitt, E. Held-Föhn, Textilveredelung 37, 19 (2003)) bestimmt werden.

### Beispiel 2

Um Cyclodextrinderivate sowohl mit proteinadsorptiven als auch biostatischen Eigenschaften herzustellen, werden folgende Cyclodextrinderivate synthetisiert.

### Umsetzung mit Guar und Triethylentetramin (Fluka)

1g Guar wird zusammen mit zwei 2g Monochlortriazenyl-β-Cyclodextrin in 200ml Wasser gelöst. Die Lösung wird auf 90°C erhitzt und es wird langsam eine Lösung von Triethylentetramin (1g/l) zugetropft, bis ein pH-Wert von 11 bis 12 eingestellt ist. Nach einer Stunde wird die Lösung abgekühlt und mit Salzsäure neutralisiert.

Nach dem Aufsprühen der Lösung auf ein Polyestergewebe wird dieses bei Raumtemperatur getrocknet. Mit Hilfe des Formazantests wird eine Hemmung des Wachstums von 80% gemessen.

Entsprechend wurden weitere proteinadsorptive Cyclodextrinderivate mit biostatischen Eigenschaften hergestellt:
wasserlösliche Stärke/Polyethylenimin (Lupasol®, BASF), Hemmung 53% Tragant/Triethylentetramin, Hemmung 82%
Carrageenan/ Triethylentetramin, Hemmung 0%
Carrageenan/Spermin (Fluka), Hemmung 10%
Guar/Diethylentriamin (Fluka), Hemmung 20%
Guar/1,2-Diaminopropan (Fluka), Hemmung 34%

### Beispiel 3

### Beispiele für die Ausrüstung textiler Materialien

A) Die in den Beispielen 1 und 2 hergestellten Lösungen werden auf ein Gewebe aus Baumwolle aufgesprüht und bei erhöhter Temperatur (40 bis 80°C) getrocknet. Die Cyclodextrine auf der Gewebeoberfläche können bei jeder Probe mit Hilfe einer basischen Phenolphthaleinlösung nachgewiesen werden. Setzt man einen Tropfen dieser violetten Lösung auf das Baumwollgewebe, dann findet eine Entfärbung durch die Komplexbildung zwischen dem Cyclodextrin und dem Phenolphthalein statt.
   Zum Nachweis der Proteinbindung wird kommerzielles Eiweißpulver verwendet. Nach einem mehrminütigen Eintauchen der Proben, die mit den in den Beispielen 1 und 2 hergestellten Lösungen eingesprüht und getrocknet worden waren, in die Eiweißlösung wird das Textil mit destilliertem Wasser vorsichtig abgespült. Danach werden die textilen Proben in verdünnte wässrige Natronlauge eingetaucht und ein paar Tropfen Kupfersulfatlösung zugesetzt. Man beobachtet eine Anfärbung der Proben, was auf die Anwesenheit von Proteinen zurückgeführt werden kann.
B) Die in den Beispielen 1 und 2 hergestellten Lösungen werden auf ein Gewebe aus Polyester aufegsprüht und getrocknet. Die Nachweise auf die Gegenwart von Cyclodextrin und die Bindung von Eiweißpulver wurden entsprechend durchgeführt und ergaben analoge Ergebnisse.
C) Anstelle von Baumwoll- und Polyestergewebe eignen sich auch Gewebe aus Polyacrylnitril, anderen synthetischen und regenerativen Fasermaterialien, Schäume aus Polyurethanen und dergleichen. Die Vorgehensweise entspricht der unter A) beschriebenen, die Ergebnisse entsprechen den dort erhaltenen Ergebnissen.

### Beispiel 4

### Cvclodextrinderivate mit reaktiven Ankergruppen

Geeignete Cyclodextrinderivate sind in der entsprechenden Patentliteratur beschrieben. Die Herstellung von Monochlortriazynsubstituiertem β-Cyclodextrin ist aus der DE 44 29 229 bekannt und weitere Cyclodextrinderivate mit einer oder mehreren reaktiven Ankergruppen aus der DE 101 55 781.

Eine Anbindung von Cyclodextrin an Fasermaterialien ist auch durch die Verwendung bifunktioneller oder polyfunktioneller Reaktanden, wie über Diisocyanate und dergleichen möglich (W. Volz, Anti-Smell-Ausrüstungen in der Textilveredelung, Textilveredelung 38, 17 (2003)), oder durch den Einsatz von Dimethylharnstoffen und ähnlichen Verbindungen, wie sie auch zur knitterfrei Ausrüstung von cellulosischen Materialien benutzt werden (H.-J. Buschmann, D. Knittel, E. Schollmeyer: Hochveredelung von Baumwolle in Anwesenheit von Cyclodextrinen zur Einlagerung von Duftstoffen, Melliand Textilbiler. 72, 198 (1991)).

### Beispiel 5

### Ausrüstung von textilen Materialien mit Cyclodextrin und Antiseptika

Als Antiseptika werden insbesondere Chlorhexidin und Polyhexanid in wässriger Lösung eingesetzt.

Drei Ausrüstungsvarianten mit Cyclodextrinderivaten, die durch ionische oder hydrophobe Wechselwirkungen auf dem Textil angebunden werden, sind gegeben:
A) Das textile Material oder der Schaum werden mit Cyclodextrinen ausgerüstet. Dazu wird eine wässrige Lösung eines Cyclodextrinderivates (s.o.) oder eine wässrige Lösung eines mit Polyvinylamin oder Polyethylenimin substituierten Cyclodextrins (ca. 3% Cyclodextringehalt) auf das auszurüstende Material aufgesprüht. Eine wässrige Lösung des Antiseptikums (0,08 bis 0,22% PHMB; 0,1 bis 0,15% Betain) wird gleichzeitig aufgesprüht, wobei beide Lösungen einen pH-Wert von 5 bis 7 haben müssen, da sonst die Bildung eines Niederschlags stattfindet.
B) Das textile Material oder der Schaum werden mit Cyclodextrin ausgerüstet. Dazu wird eine wässrige Lösung eines Cyclodextrinderivates (s.o.) oder eine wässrige Lösung eines Polyvinylamin oder Polyethylenimin substituierten Cyclodextrins (ca. 3% Cyclodextringehalt) bei einem pH-Wert von 5 bis 7 auf das auszurüstende Material aufgesprüht und getrocknet. Anschließend wird eine wässrige Lösung des Antiseptikums (0,1% PHMB; 0,1% Betain) aufgesprüht. Der Auftrag beider Lösungen kann auch mit Hilfe eines Foulards durchgeführt werden.
C) Eine Mischung des Cyclodextrinderivates (s.o.) oder eines mit Polyvinylamin bzw. Polyethylenimin substituierten Cyclodextrins und des Antiseptikums wird durch das Mischen beider Komponenten (equimolar; bevorzugt 0,001 bis 0,01mol/l) in wässriger Lösung hergestellt, wobei die wässrige Phase einen pH-Wert von 5 bis 7 besitzen muss, um die Bildung eines unlöslichen Niederschlags zu verhindern. Die resultierende wässrige Lösung wird auf das Textilmaterial aufgesprüht oder durch einen Auftrag mit Hilfe eines Foulards auf das textile Material oder den Schaum aufgebracht.

### Beispiel 6

### Ausrüstungsvariante mit einem Cyclodextrinderivat mit einer reaktiven Gruppe

Ein Baumwollgewebe wird in eine wässrige Lösung von Monochlortriazinyl-β-Cyclodextrin (Natriumsalz, 20-100g/l) (Cavasol® W7 MCT, Wacker-Chemie GmbH) eingetaucht, die mit Essigsäure (60%) auf einen pH-Wert von 4 bis 6 eingesetzt ist. Anschließend wird das Gewebe auf einen Foulard abgequetscht (Flottenaufnahme ca. 70%). Danach wird das Gewebe bei 80°C für 10 Minuten vorgetrocknet und anschließend bei 160°C für 7 Minuten fixiert. Anschließend wird eine wässrige Lösung des Antiseptikums (0,1% PHMB) aufgesprüht oder durch einen Auftrag mit Hilfe eines Foulards aufgebracht. Gleichzeitig oder anschließend wird das Betain aus wässriger Lösung (0,1% Betain) aufgebracht.

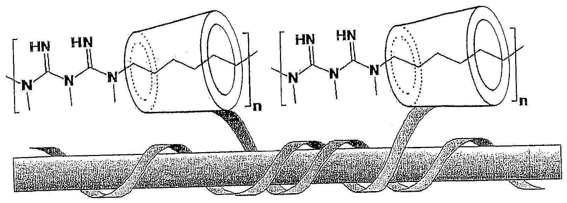

Die Abbildung zeigt schematisch eine Cyclodextrinmolekül mit Polyhexamidbeladung und einer Ankergruppe, die das Cyclodextrin mechanisch an einer Textilfaser verankert.

## Patentansprüche

1. Wundauflage enthaltend ein Trägermaterial und wenigstens ein Antiseptikum zusammen mit einem Tensid, **dadurch gekennzeichnet, dass** das Trägermaterial mit einem Cyclodextrin oder Cyclodextrinderivat ausgerüstet ist, das mit dem Antiseptikum beladen ist, und das Tensid ein Betaïn ist, wobei das Cyclodextrin oder Cyclodextrinderivat chemisch an ein Polysaccharid, ein Polyvinylamin oder ein Polyethylenimin gebunden ist, das seinerseits über physikalische Wechselwirkungen an das Trägermaterial gebunden ist.

2. Wundauflage nach Anspruch 1, **dadurch gekennzeichnet, dass** das Trägermaterial ein polymerer Schaum ist, insbesondere ein poröser Polyurethanschaum.

3. Wundauflage nach Anspruch 1, **dadurch gekennzeichnet, dass** das Trägermaterial ein textiles Trägermaterial ist, insbesondere ein Polyester oder Baumwolle.

4. Wundauflage nach einem der vorstehenden Ansprüche, **dadurch gekennzeichnet, dass** das Trägermaterial mit einem β-Cyclodextrin oder einem β-Cyclodextrinderivat ausgerüstet ist.

5. Wundauflage nach einem der vorstehenden Ansprüche, **gekennzeichnet durch** einen Gehalt von 1 bis 5 Gew.-% Cyclodextrin oder Cyclodextrinderivat im Trägermaterial, bezogen auf das Gewicht des Trägermaterials.

6. Wundauflage nach einem der vorstehenden Ansprüche, **dadurch gekennzeichnet, dass** das Antiseptikum eines aus der Gruppe der Polyamine oder Biguanide sind.

7. Wundauflage nach Anspruch 6, **dadurch gekennzeichnet, dass** das Cyclodextrin oder Cyclodextrinderivat mit Chlorhexidin oder Polyhexanid beladen ist.

8. Wundauflage nach einem der vorstehenden Ansprüche, **dadurch gekennzeichnet, dass** das molekulare Verhältnis Cyclodextrin oder Cyclodextrinderivat zu Antiseptikum im Bereich von 0,1 bis 1,0 liegt.

9. Wundauflage nach einem der vorstehenden Ansprüche, **dadurch gekennzeichnet, dass** das Betaïn Undecylenamidopropylbetaïn oder Trimethylammoniumacetat ist.

10. Wundauflage nach einem der vorstehenden Ansprüche, **dadurch gekennzeichnet, dass** es Antiseptikum und Betaïn in etwa äquimolaren Mengen enthält.

11. Verwendung eines Polyvinylamin-ß-Cyclodextrins mit Polyhexamid-Beladung in wässriger Lösung zusammen mit einem Betaïn zur Herstellung von antiseptischen Wundauflagen.

## Claims

1. A wound dressing containing a carrier material and at least one antiseptic together with a surfactant, **characterised in that** the carrier material is treated with a cyclodextrin or cyclodextrin derivative which is loaded with the antiseptic, and the surfactant is a betaine, wherein the cyclodextrin or cyclodextrin derivative is chemically bound to a polysaccharide, a polyvinyl amine or a polyethyleneimine, which for its part is bound to the carrier material via physical interactions.

2. The wound dressing according to claim 1, **characterised in that** the carrier material is a polymeric foam, in particular a porous polyurethane foam.

3. The wound dressing according to claim 1, **characterised in that** the carrier material is a textile carrier material, in particular a polyester or cotton.

4. The wound dressing according to one of the preceding claims, **characterised in that** the carrier material is treated with a β-cydodextrin or a β-cyclodextrin derivative.

5. The wound dressing according to one of the preceding claims, **characterised by** a content of 1 to 5 wt.% of cyclodextrin or cyclodextrin derivative in the carrier material, based on the weight of the carrier material.

6. The wound dressing according to one of the preceding claims, **characterised in that** the antiseptic is one from the group of the polyamines or biguanides.

7. The wound dressing according to claim 6, **characterised in that** the cyclodextrin or cyclodextrin derivative is loaded with chlorhexidine or polyhexanide.

8. The wound dressing according to one of the preceding claims, **characterised in that** the molecular ratio of cyclodextrin or cyclodextrin derivative to antiseptic lies in the range of 0.1 to 1.0.

9. The wound dressing according to one of the preceding claims, **characterised in that** the betaine is undecylenamidopropyl betaine or trimethylammonium acetate.

10. The wound dressing according to one of the preceding claims, **characterised in that** it contains antiseptic and betaine in approximately equimolar amounts.

11. Use of a polyvinylamine-β-cyclodextrin with polyhexamide loading in aqueous solution together with a betaine for the preparation of antiseptic wound dressings.

## Revendications

1. Pansement contenant un matériau support et au moins un antiseptique avec un tensioactif, **caractérisé en ce que** le matériau support est apprêté avec une cyclodextrine ou un dérivé de cyclodextrine, qui est chargée avec l'antiseptique, et le tensioactif est une bétaïne, la cyclodextrine ou le dérivé de cyclodextrine étant liée chimiquement à un polysaccharide, une polyvinylamine ou une polyéthylèneimine, qui, de son côté, est lié(e) au matériau support par le biais d'interactions physiques.

2. Pansement selon la revendication 1, **caractérisé en ce que** le matériau support est une mousse polymère, en particulier une mousse de polyuréthane poreuse.

3. Pansement selon la revendication 1, **caractérisé en ce que** le matériau support est un matériau support textile, en particulier un polyester ou du coton.

4. Pansement selon l'une des revendications précédentes, **caractérisé en ce que** le matériau support est apprêté avec une β-cyclodextrine ou un dérivé de β-cyclodextrine.

5. Pansement selon l'une des revendications précédentes, **caractérisé par** une teneur de 1 à 5 % en poids en cyclodextrine ou dérivé de cyclodextrine dans le matériau support, par rapport au poids du matériau support.

6. Pansement selon l'une des revendications précédentes, **caractérisé en ce que** l'antiseptique est un antiseptique du groupe des polyamines ou biguanides.

7. Pansement selon la revendication 6, **caractérisé en ce que** la cyclodextrine ou le dérivé de cyclodextrine est chargé(e) avec de la chlorhexidine ou du polyhexanide.

8. Pansement selon l'une des revendications précédentes, **caractérisé en ce que** le rapport moléculaire entre la cyclodextrine ou le dérivé de cyclodextrine et l'antiseptique est compris entre 0,1 et 1,0.

9. Pansement selon l'une des revendications précédentes, **caractérisé en ce que** la bétaïne est de l'undecylenamidopropyl bétaïne ou du triméthylammonium acétate.

10. Pansement selon l'une des revendications précédentes, **caractérisé en ce qu'**il contient de l'antiseptique et de la bétaïne en quantités approximativement équimolaires.

11. Utilisation d'une polyvinylamine-β-cyclodextrine avec une charge de polyhexamide dans une solution aqueuse, conjointement avec une bétaïne pour la fabrication de pansements antiseptiques.
